# EUROPEAN PATENT SPECIFICATION

(11) **EP 2 942 074 B1**
(45) Date of publication and mention of the grant of the patent: **03.01.2018**
(21) Application number: 14735368.4
(22) Date of filing: 03.01.2014
(51) Int. Cl.: A61M 5/32

(54) **MACHINE FOR RECYCLING SHARP, POINTED OBJECTS**
MASCHINE ZUM RECYCLING VON SCHARFEN, SPITZEN GEGENSTÄNDEN
MACHINE DE RECYCLAGE D'OBJETS TRANCHANTS

(30) Priority: 03.01.2013 ES 201330004
(43) Date of publication of application: 11.11.2015
(73) Proprietor: Recysclinic, S.L.U., 18200 Albolote (Granada) (ES)
(72) Inventor: SALAS BURRUEZO, Manuel, 18220 Albolote (Granada) (ES)
(74) Representative: Munoz Garcia, Antonio
(86) International application number: PCT/ES2014/070002
(87) International publication number: WO 2014/106679

(56) References cited:
- EP-A1- 1 447 107
- WO-A1-91/03984
- ES-T3- 2 095 652
- FR-A1- 2 773 465
- KR-B1- 101 205 653
- US-A- 4 315 592
- US-A- 5 351 381
- US-A- 5 857 569
- US-A1- 2008 217 447

## Description

### Object of the Invention

As expressed in the title of the present specification, the invention relates to a machine for recycling sharp, pointed objects which provides innovative features and advantages for its intended function, which features and advantages will be described below and entail a significant novelty in the current state of the art.

More particularly, the object of the invention is centered on a machine the purpose of which is to allow separating, in a fast, simple and safe manner, the metal portion of sharp, pointed objects, such as syringes, scalpels or other instruments used in hospitals, clinics, health centers, etc., thereby allowing recycling thereof and preventing accidents.

### Field of Application of the Invention

The field of application of the present invention is encompassed within the sector of the industry dedicated to manufacturing recycling systems, devices and apparatus, focusing particularly on those intended for the health field.

### Background of the Invention

It is known that waste generated in healthcare centers, such as, for example, hospitals, must be recycled. This, however, is not easy in cases such as syringes, scalpels, some tools used in dentistry, and generally any type of sharp, pointed object. The reason is that these elements can have portions made of different materials, or recyclable and non-recyclable portions that are firmly attached to one another. For example, syringes are formed by a plastic reservoir attached to a metal needle; scalpels can have a reusable handle attached to a disposable blade, etc.

Today, in these cases the entire part is thrown into yellow containers distributed for such purpose by the hospital so that they can subsequently then be discarded. The economic loss and environmental damage entailed by this treatment is easily understood. The document ES2095652T discloses the features of the preamble of claim 1. The patent document EP1447107A1 discloses a device for destroying sharp, pointed objects which, although it does share some general features with the invention herein proposed, incorporates a fairly complex system comprising an unscrewing mechanism and another needle melting mechanism, so it has the drawback of being a very expensive apparatus to make.

Also, patent document US5857569 discloses a needle extraction device and system by means of twisting basically comprising different-shaped slots in which the needle is inserted for breaking and extracting it. However, unlike the machine of the present invention, it does not separate the metal part of the needle from its plastic part, so it does not allow suitable material recycling.

There are also other documents disclosing systems relating to waste recycling, but they do not have features in common with the machine of the invention as it is claimed, for example patent document US4315592A relating to a receptacle for syringes which is completely obsolete as it does not comply with regulations since it is a container for depositing the syringes with the needles still inserted that is made from corrugated cardboard that can be taken apart, while today it is necessary to use rigid and officially approved receptacles for waste of this type.

### Brief Description of the Invention

The present invention solves the described problems as a result of a machine particularly designed for separating the hypodermic needle from the plastic reservoir in syringes, and the handle from the blade in scalpels. This allows recycling, on one hand, plastic materials (plastic reservoirs of syringes, the handle of single-use scalpels, etc.) and, on the other hand, metal materials (syringe needles, scalpel blades).

A significant advantage of this invention relates to the reduction of occupational accidents with sharp, pointed objects, since the risk is eliminated at the same time in which it is created.

Furthermore, there is a need to mention the obvious environmental benefits of recycling materials that were previously discarded, also the amount of recipients for sharp objects the manufacture thereof could be avoided, as well as the improvement in the income obtained as a result of recycling these elements, in addition to the fact that it is no longer necessary to pay a waste management company to dispose of sharp, pointed objects, since with this system the waste can be sterilized in an autoclave separately in the same center for subsequent recycling. The economic benefit is therefore doubled.

The machine for recycling sharp, pointed objects of the present invention is defined by the features of claim 1. It must be pointed out that although the invention can be implemented in the most essential form with a single spout for syringes, in a preferred embodiment, two spouts of different sizes are arranged, one for large syringes and another for small syringes. An opening for disassembling both single-use and multi-use scalpel blades is also contemplated in the upper portion of the machine. It consists of an H-shaped opening with a metal tongue adapted underneath same, this tongue being structured for holding the scalpel blade when rotating it, such that when introducing the scalpel through the upper portion of the opening, the blade is moved from its housing, making it rotate slightly, and by pulling the handle of the scalpel upwards, as the scalpel is held in the tongue, it is separated from the handle and falls into the metallic waste reservoir.

Optionally, the machine furthermore has an additional freely accessible opening, preferably provided with a closure lid, and the size of which allows introducing other metallic waste generated in hospitals and medical centers that do not require the separation of non-metal portions. Some small metal tools such as drills used in dentistry or suture needles used in other fields can be thrown away through this freely accessible opening, for example. The syringe needle extraction system consists of a pair of gear wheels meshing with one another and arranged below each spout for syringes.

Each pair of gear wheels which are moved by an electric motor catch and take the needle out of the syringe introduced through the spout while rotating.

To that end, the hollow casing has therein a motor and a battery or a connection to the system, furthermore having a start push-button, a connector for charging the battery and a three-position switch, i.e., left, right and stop positions, for rotating the shaft of the motor in one direction or another.

Therefore, when the user introduces the hypodermic needle of a syringe through the suitable spout, the hand holding the syringe is supported on the push-button which the machine has in the upper portion and the pair of gear wheels engages the needle and pulls it downwards. Since the spout has a size slightly smaller than the diameter of the body of the syringe, the latter is immobilized therein. As a result, the gear wheels take the needle out of the reservoir, the needle falling through the ramp that allows access to the metallic waste reservoir.

The electric motor used for operating the gears can be a DC motor, in which case it would be connected to a 12V DC battery. This configuration would allow implementing the machine of the invention in moving structures, such as for example a trolley for medical or clinical use. However, in another preferred embodiment of the invention, an AC motor is used, in which case it would be connected to the 220V AC power grid. The syringe needle extraction system provided below the spouts may comprise other alternatives, not falling under the scope of the present invention, such as the incorporation in the machine of other alternative extraction systems for extraction using pressure, rotation, absorption etc., and operated by means of an electric motor or by means of a manual mechanism, is not ruled out.

In any case, the casing contemplates below the needle extraction system a lower access surface for accessing the metal object reservoir, which is removable so that it can be emptied.

This lower surface is configured like a box with an access ramp, and to prevent the metallic waste from being able to come out when extracting the reservoir, the latter is provided with a sliding lid which is coupled to guides provided in the lower portion of said box with a ramp below which it is located.

Therefore, the sliding lid is coupled by means of guides to the upper portion of the reservoir and is fixed thereto by a screw that it has in the upper portion, or by any other system. The reservoir is in turn fixed to the box by means of a magnet that the latter has in the inner wall, for example.

It is thereby assured that the reservoir is always open so that the waste falls into it, and when it is extracted, the sliding lid thereof closes to prevent waste from coming out from inside the reservoir.

The reservoir stores the needles taken out of the syringes, the blades disassembled from the scalpels and other metallic waste that may have optionally been introduced through the additional opening. This reservoir will contain only metal parts, and therefore recycling is possible without needing subsequent complex and costly separations.

It must be mentioned that the reservoir does not need any fill detector, since it will be sized to have sufficient capacity for storing needles up to six months, as required by biological waste regulations. However, if this were not the case in some cases, it can further comprise a fill detector located close to the upper edge thereof, for example, an ultrasound detector. This detector will be able to activate a visual alarm, such as a light indicator, for example, a LED, indicating that the reservoir is full, or it can simply be a metal detector which, when it detects the metallic waste, blocks the operation of the machine and makes the replacement thereof necessary.

Therefore, the waste generated, for example, after a surgery can be disposed of in a fast, simple and safe manner. Introducing each syringe used in the corresponding spout will be sufficient, the machine of the invention being responsible for taking out the metal needles and storing them together with the metallic waste. The user will then throw the plastic body of the syringe into the plastic container.

Similarly, the user introducing the scalpel blade used in said operation through the corresponding H-shaped opening and then slightly rotating the blade inserted therein will be sufficient. By pulling on the handle, the blade is locked in the tongue, being taken out of the handle, and it will be stored in the reservoir of the machine.

The described machine for recycling sharp, pointed objects therefore represents an innovative structure having structural and constitutive features that were unknown up until now for this purpose, and these reasons combined with its practical usefulness give the invention sufficient grounds for obtaining the exclusive right that is sought.

### Description of the Drawings

To complement the description that is being made and for the purpose of aiding to better understand the features of the invention, a set of drawings is attached to the present specification as an integral part thereof, in which the following has been depicted with an illustrative and nonlimiting character:
Figure 1 shows an exploded perspective view of the machine for recycling sharp, pointed objects, object of the invention, the main portions and elements it comprises being seen therein.
Figure 2 shows an enlarged view of the system for separating scalpel blades incorporated in the machine according to the invention.

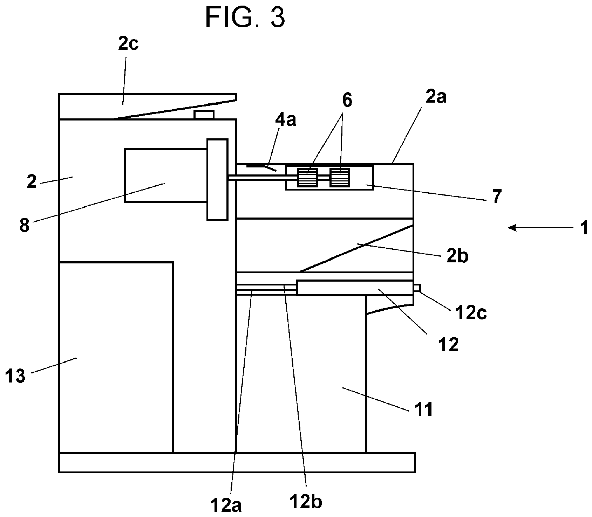

Figure 3 shows a sectioned side elevational view of the example of the machine according to the invention shown in Figure 1, being depicted once assembled.

### Preferred Embodiment of the Invention

In view of the mentioned drawings and according to the numbering used, the invention which comprises the portions and elements indicated and described in detail below can be seen therein.

Therefore, in reference to said drawings, in which an example of the machine of the present invention is shown, it can be seen how the machine (1) at hand comprises a casing (2) having an essentially planar upper front metal surface (2a), below which there is coupled a box (2b) with a surface in the form of a ramp that allows access to a reservoir (11) located therebelow.

The upper surface (2a) has one or more spouts (3) having suitable diameters for introducing the needles of the syringes to be disposed of, as well as at least one H-shaped opening (4) associated with means for disassembling the scalpel blade and optionally at least one additional freely accessible opening (5) with a lid, for introducing other metallic waste, such as some tools such as drills, suture needles, etc.

The machine has associated with each spout (3) an extraction system for extracting the mentioned needles for separating them from their body which, consists of respective pairs of gear wheels (6) meshing with one another and held by a support (7). The pairs of gear wheels (6) are located in line immediately below each spout (3) and meshing with one another, such that all of them are moved when the electric motor (8) provided in the casing (1) for such purpose operates the drive gear wheel which is associated with the shaft thereof, by means of screwing or by another fastening system. Therefore, it is not necessary to use any movement transmission mechanism for operating all the gear wheels (6), the electric motor (8) being able to be located in the hollow rear portion of the casing (2) of the machine (1), which can be accessed for quicker repair from a practicable lid (not shown).

There has been provided in the upper portion of the casing (2) a push-button (2c) for starting up the motor (8) and operating the gear mechanism.

On the other hand, there has been provided a three-position switch (9) allowing passage of the power supply and reversing the direction of rotation of the motor (8) and thereby causing the gears (6) to be released and the support (7) in which they are coupled to be extracted for easy cleaning.

The electric motor (8) is powered by means of a 12V DC battery (13) which can be recharged by plugging a battery charger into a plug (10), although it can also be directly connected to the 220V AC grid.

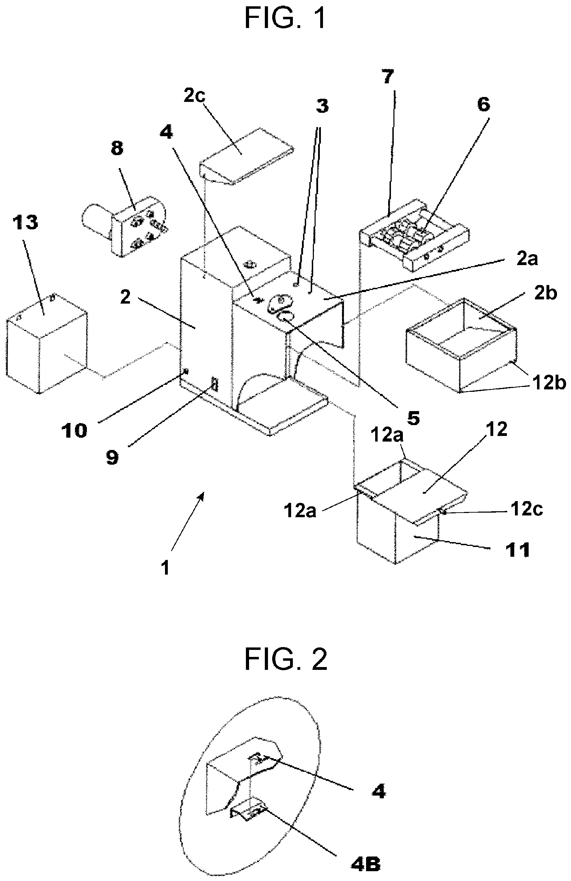

In reference to Figure 2, it can be observed how in the H-shaped opening (4) incorporated in the machine for disassembling scalpel blades, there has been provided a metal tongue (4b) adapted therebelow and the configuration of which allows holding the blades such that when introducing the scalpel through the upper portion of the H-shaped opening (4), the blade is moved from its housing, and by pulling the handle of the scalpel upwards, it is immobilized in the tongue, falling into the metallic objects reservoir (11).

The casing (2) is preferably formed by a rear body in which, where appropriate, the motor and the battery are housed, and a front part with an open front the upper portion of which constitutes the planar upper surface in which the spouts for the syringe needles , the H-shaped opening for the scalpel blades and the additional freely accessible opening are located, the lower portion of said front part of the machine (1) being taken up by the reservoir (11) into which all the syringe needles, scalpel blades and other metal tools introduced in the machine (1) fall.

This reservoir has the particularity of having a sliding lid (12) which, when inserted in the respective guides (12a) provided in the upper portion of the reservoir (11), also in turn enters other complementary guides (12b) of the box (2b) with a ramp below which there is located the reservoir, such that when the reservoir is introduced below said box (2b), by pushing it inwards the sliding lid opens, leaving the passage open for the waste to fall therein, and when the reservoir is extracted, the sliding lid closes so that the infected waste cannot be accessed until it is sterilized in an autoclave or any sterilization system. To fix said sliding lid (12) in its closed position, a closing system consisting, for example, of a screw (12c) is contemplated.

Having sufficiently described the nature of the present invention as well as the manner of putting it into practice, it is not considered necessary to further describe the invention so that a person skilled in the art can comprehend the scope thereof and advantages derived from it, stating that it could be carried out to practice within its essential features in other embodiments limited by the scope of the appended claims.

## Claims

1. A machine for recycling sharp, pointed objects, comprising a casing (2) with an upper front metal surface (2a) below which there is coupled a box (2b) with a surface in the form of a ramp that allows access to a reservoir (11) located therebelow; wherein said front metal surface (2a) has at least one spout (3) suitable for introducing syringe needles, associated with a needle extraction system for separating said needles from the plastic body of a syringe, and at least one opening (4), **characterized in that** the needle extraction system associated with the at least one spout (3) consists of pairs of gear wheels (6) meshing with one another, which are held by a support (7) and are moved by an electric motor (8), which gear wheels (6) catch and take the needle out of the syringe introduced through the at least one spout while rotating, separating it from the plastic body of the syringe and **in that** the opening is an H-shaped opening (4) suitable for introducing scalpel blades and is associated with means for disassembling said scalpel blades wherein the machine incorporates below the H-shaped opening (4) a metal tongue (4b) adapted therebelow configured for holding the scalpel blades when making them rotate in said H-shaped opening (4).

2. The machine for recycling sharp, pointed objects according to claim 1, **characterized in that** it comprises two spouts (3) with different diameters for introducing needles of different sizes.

3. The machine for recycling sharp, pointed objects according to claim 1 or 2, **characterized in that** the gear wheels (6) are located in line and meshing with one another, such that all of them are moved when the electric motor (8) operates the drive gear wheel (6) which is associated with the shaft of the same motor (8) by means of screwing or another fastening system.

4. The machine for recycling sharp, pointed objects according to claim 3, **characterized in that** there has been provided in the casing (2) a push-button (2c) for starting up the motor (8) and operating the gear mechanism.

5. The machine for recycling sharp, pointed objects according to any of claims 3-4, **characterized in that** a three-position switch (9) allowing passage of the power supply and reversing the direction of rotation of the motor (8) in order to release the gears (6) and to be able to extract the support (7) has been provided.

6. The machine for recycling sharp, pointed objects according to any of claims 3-5, **characterized in that** it comprises a 12V DC rechargeable battery (13) for powering the electric motor (8).

7. The machine for recycling sharp, pointed objects according to any of claims 3-5, **characterized in that** it is directly connected to the 220V AC grid for powering the electric motor (8).

8. The machine for recycling sharp, pointed objects according to any of claims 1-7, **characterized in that** the reservoir (11) has a sliding lid (12) which, when inserted in the respective guides (12a) provided in the upper portion of the reservoir (11), also in turn enters the complementary guides (12b) provided to that end in the box (2b) with a ramp below which the reservoir is located.

9. The machine for recycling sharp, pointed objects according to any of claims 1-8, **characterized in that** there has been provided on the front metal surface (2a) of the casing (2) at least one additional freely accessible opening (5) with a lid for introducing other metallic waste.

## Patentansprüche

1. Maschine zum Recycling von scharfen, spitzen Gegenständen, umfassend ein Gehäuse (2) mit einer oberen metallischen Stirnfläche (2a), unter welcher ein Kasten (2b) mit einer Fläche in Form einer Rampe gekoppelt ist, welche den Zugang zu einem sich darunter befindenden Sammelbehälter (11) erlaubt; wobei die genannte metallische Stirnfläche (2a) mindestens eine Tülle (3), welche dazu geeignet ist, Injektionsnadeln einzuführen und mit einem Nadelextraktionssystem zum Trennen der genannten Nadeln aus dem Kunststoffkörper einer Spritze assoziiert ist, und mindestens eine Öffnung (4) aufweist, **dadurch gekennzeichnet, dass** das mit der mindestens einen Tülle (3) assoziierte Nadelextraktionssystem aus einem Paar von Zahnrädern (6), die ineinandergreifen, besteht, welche von einer Stütze (7) gehalten werden und von einem Elektromotor (8) bewegt werden, welche Zahnräder (6) die Nadel greifen und aus der durch die mindestens eine Tülle eingeführte Spritze beim Drehen entnehmen, so dass sie vom Kunststoffkörper der Spritze getrennt wird und dass die Öffnung eine H-förmige Öffnung (4) ist, welche dazu geeignet ist, Skalpelklingen einzuführen und mit Mitteln zum Demontieren der genannten Skalpelklingen assoziiert ist, wobei in der Maschine unterhalb der H-förmigen Öffnung (4) eine Metallzunge (4b) eingebaut ist, welche darunter angepasst ist und dazu ausgebildet ist, die Skalpelklingen zu halten, wenn sie in der genannten H-förmigen Öffnung (4) gedreht werden.

2. Maschine zum Recycling von scharfen, spitzen Gegenständen nach Anspruch 1, **dadurch gekennzeichnet, dass** sie zwei Tüllen (3) mit unterschiedlichen Durchmessern zum Einführen von Nadeln mit unterschiedlichen Größen umfasst.

3. Maschine zum Recycling von scharfen, spitzen Gegenständen nach Anspruch 1 oder 2, **dadurch gekennzeichnet, dass** sich die Zahnräder (6) in Reihe und ineinandergreifend befinden, so dass alle bewegt werden, wenn der Elektromotor (8) das Antriebszahnrad (6) betätigt, welches mit der Welle des gleichen Motors (8) mittels Verschraubung oder eines anderen Befestigungssystems assoziiert ist.

4. Maschine zum Recycling von scharfen, spitzen Gegenständen nach Anspruch 3, **dadurch gekennzeichnet, dass** im Gehäuse (2) ein Druckknopf (2c) zum Inbetriebsetzen des Motors (8) und zum Betreiben des Zahnradgetriebes bereitgestellt worden ist.

5. Maschine zum Recycling von scharfen, spitzen Gegenständen nach einem der Ansprüche 3-4, **dadurch gekennzeichnet, dass** ein Dreistellungsschalter (9) bereitgestellt worden ist, welcher den Durchgang der Stromversorgung und die Umkehr der Drehrichtung des Motors (8) erlaubt, um die Getriebe (6) zu lösen und in der Lage zu sein, die Stütze (7) herauszunehmen.

6. Maschine zum Recycling von scharfen, spitzen Gegenständen nach einem der Ansprüche 3-5, **dadurch gekennzeichnet, dass** sie eine 12V wiederaufladbare Gleichstrombatterie (13) zum Versorgen des Elektromotors (8) umfasst.

7. Maschine zum Recycling von scharfen, spitzen Gegenständen nach einem der Ansprüche 3-5, **dadurch gekennzeichnet, dass** sie direkt mit dem 220V Wechselstromnetz zum Versorgen des Elektromotors (8) verbunden ist.

8. Maschine zum Recycling von scharfen, spitzen Gegenständen nach einem der Ansprüche 1-7, **dadurch gekennzeichnet, dass** der Sammelbehälter (11) einen Schiebedeckel (12) aufweist, welcher, wenn in die jeweiligen Führungen (12a) eingesteckt wird, welche im oberen Teil des Sammelbehälters (11) bereitgestellt werden, wiederum auch in die komplementären Führungen (12b) eindringt, welche zu diesem Zweck im Kasten (2b) mit einer Rampe bereitgestellt werden, unter welchem sich der Sammelbehälter befindet.

9. Maschine zum Recycling von scharfen, spitzen Gegenständen nach einem der Ansprüche 1-8, **dadurch gekennzeichnet, dass** auf der metallischen Stirnfläche (2a) des Gehäuses (2) mindestens eine zusätzliche freizugängliche Öffnung (5) mit einem Deckel zum Einführen eines anderen Metallabfalls bereitgestellt worden ist.

## Revendications

1. Machine pour recycler des objets tranchants, pointus, comprenant un boîtier (2) avec une surface métallique supérieure avant (2a) sous laquelle se trouve couplée une boîte (2b) avec une surface en forme de rampe qui permet l'accès à un réservoir (11) situé sous celle-ci; dans laquelle ladite surface métallique avant (2a) a au moins un embout (3) apte à introduire des aiguilles de seringue, associées a un système d'extraction d'aiguilles pour séparer lesdites aiguilles du corps en plastique d'une seringue, et au moins une ouverture (4), **caractérisée en ce que** le système d'extraction d'aiguilles associé à l'au moins un embout (3) consiste à des paires de roues d'engrenages (6) mutuellement en prise, qui sont maintenues par un support (7) et sont déplacées par un moteur électrique (8), lesdites roues d'engrenages (6) attrapent et sortent l'aiguille de la seringue introduite à travers l'au moins un embout pendant qu'il tourne, en le séparant du corps en plastique de la seringue et **en ce que** l'ouverture est une ouverture en forme de H (4) apte à introduire des lames de bistouri et est associée à des moyens de démontage desdites lames de bistouri dans laquelle la machine incorpore sous l'ouverture en forme de H (4) une languette métallique (4b) adaptée sous celle-ci pour soutenir les lames de bistouri lorsqu'elle les fait tourner dans ladite ouverture en forme de H (4).

2. Machine pour recycler des objets tranchants, pointus selon la revendication 1, **caractérisée en ce qu'**elle comprend deux embouts (3) avec différents diamètres pour introduire des aiguilles de différentes tailles.

3. Machine pour recycler des objets tranchants, pointus selon la revendication 1 ou 2, **caractérisée en ce que** les roues d'engrenages (6) sont situées en ligne et mutuellement en prise, de manière que toutes celles-ci sont déplacées lorsque le moteur électrique (8) met en marche la roue d'engrenages d'entraînement (6) qui est associée à l'arbre du même moteur (8) par le biais d'un système de vissage ou d'un autre système de fixation.

4. Machine pour recycler des objets tranchants, pointus selon la revendication 3, **caractérisée en ce que** dans le boîtier (2) est prévu un bouton poussoir (2c) pour démarrer le moteur (8) et mettre en marche le mécanisme d'engrenages.

5. Machine pour recycler des objets tranchants, pointus selon l'une quelconque des revendications 3-4, **caractérisée en ce qu'**on a prévu un commutateur à trois positions (9) qui permet le passage de la source d'alimentation électriques et l'inversion de la direction de rotation du moteur (8) afin de libérer les engrenages (6) et pouvoir extraire le support (7).

6. Machine pour recycler des objets tranchants, pointus selon l'une quelconque des revendications 3-5, **caractérisée en ce qu'**elle comprend une batterie rechargeable de 12V CC (13) pour alimenter le moteur électrique (8).

7. Machine pour recycler des objets tranchants, pointus selon l'une quelconque des revendications 3-5, **caractérisée en ce qu'**elle est directement connectée au réseau de 220 V CA pour alimenter le moteur électrique (8).

8. Machine pour recycler des objets tranchants, pointus selon l'une quelconque des revendications 1-7, **caractérisée en ce que** le réservoir (11) a un couvercle coulissant (12) qui, lorsqu'il est inséré dans les guides (12a) respectives prévues dans la portion supérieure du réservoir (11), rentre également à son tour dans les guides complémentaires (12b) prévues à cette fin dans la boîte (2b) avec une rampe sous laquelle est situé le réservoir.

9. Machine pour recycler des objets tranchants, pointus selon l'une quelconque des revendications 1-8, **caractérisée en ce que** sur la surface métallique avant (2a) du boîtier (2) est prévue au moins une ouverture supplémentaire librement accessible (5) avec un couvercle pour introduire d'autre déchet métallique.
